**Europäisches Patentamt**

**European Patent Office**

**Office européen des brevets**

(11) Veröffentlichungsnummer: **0 144 699 B1**

## EUROPÄISCHE PATENTSCHRIFT

(45) Veröffentlichungstag der Patentschrift: **13.03.91**

(51) Int. Cl.⁵: **A61F 5/44**

(21) Anmeldenummer: **84112991.9**

(22) Anmeldetag: **27.10.84**

(54) Vorrichtung zum wahlweisen Öffnen und Verschliessen von rohrförmigen Körperorganen, insbesondere der Harnröhre.

(30) Priorität: **02.11.83 DE 8331338 U**

(43) Veröffentlichungstag der Anmeldung:
**19.06.85 Patentblatt 85/25**

(45) Bekanntmachung des Hinweises auf die
Patenterteilung:
**13.03.91 Patentblatt 91/11**

(84) Benannte Vertragsstaaten:
**DE FR GB SE**

(56) Entgegenhaltungen:
**EP-A- 0 093 507**
**CH-A- 463 015**
**US-A- 4 386 601**

(73) Patentinhaber: **ESKA medical & kunststoff-technik gmbh & co., vormals Walter Koss**
**Industriestrasse 2 Postfach 1164**
**W-6222 Geisenheim am Rhein(DE)**

(72) Erfinder: **Jonas, Udo, Prof. Dr.**
**Oranje Nassaulaan 31**
**NL-2361 LB Warmond(NL)**
Erfinder: **Koss, Walter**
**Industriestrasse**
**W-6222 Geisenheim(DE)**

(74) Vertreter: **Blumbach Weser Bergen Kramer**
**Zwirner Hoffmann Patentanwälte**
**Sonnenberger Strasse 100**
**W-6200 Wiesbaden(DE)**

## Beschreibung

Die Erfindung betrifft eine implantierbare Vorrichtung nach dem Oberbegriff des Anspruchs 1.

Vorrichtingen der vorgenannten Art sind insbesondere in Form von Prothesen zur Behandlung der Harninkontinenz bekannt. Dabei handelt es sich um Verschlüsse, die auf die Harnröhre einwirken und beim Versagen des natürlichen Schließmuskels dessen Funktion übernehmen. Bekannt ist beispielsweise eine implantierbare Harninkontinenzprothese, die kissenartig aus Siliconkautschuk und Silicongel hergestellt ist und schwammartig so auf die Harnröhre drückt, daß diese verschlossen ist, so lange kein größerer Druck auftritt. Es wird dabei also nur der Ausflußwiderstand vergrößert, so daß die Blase zur Entleerung jeweils den Widerstand überwinden muß. Der Durchlaß kann nicht willkürlich geöffnet und geschlossen werden. Bekannt ist auch eine implantierbare Prothese, bei der eine mittels einer Flüssigkeit aufpumpbare Manschette am Blasenausgang um die Harnröhre gelegt wird. Zusätzlich werden zwei kleine, durch die Haut bedienbare Pumpen sowie ein Flüssigkeitsreservoir implantiert. Mit der einen Pumpe kann die Manschette gefüllt und damit in die Schließstellung gebracht und mit der anderen unter Transport der Druckflüssigkeit in das Reservoir entleert werden. Sowohl der Aufwand für die Prothese selbst als auch der Aufwand für das Implantieren sind hoch. Die verschiedenen Bestandteile mit ihren verhältnismäßig komplizierten Elementen vergrößern die Gefahr eines Versagens, wodurch dann Reparaturen mit operativem Eingriff nötig werden.

Bekannt ist aus der CH-A-463 015 außerdem eine implantierbare Vorrichtung zum wahlweisen Öffnung und Verschließen der Harnröhre, bei der gegeneinander bewegbare Klemmteile für das Verschließen der Harnröhre vorgesehen sind. Die Klemmteile stehen unter der Einwirkung einer Feder und eines Magneten. Sie können gegeneinander gleiten, sind aber nicht verformbar.

Schließlich ist eine Prothese für die männliche Harninkontinenz bekannt (DE-GU 79 28 052), bei der eine die Harnröhre umschließende Manschette durch Druckausübung einen Verschluß der Harnröhre ermöglicht. Die Manschette weist eine spaltförmige Durchgangsöffnung für das Organ auf und ist wenigstens in einem Teilbereich so ausgebildet, daß dieser Bereich durch rückfederungsarmes Biegen in eine Verschließ- bzw. Öffnungsstellung bringbar ist. Die Manschette besteht vorzugsweise aus implantierbarem Kunststoff mit einer Einlage aus biegeweichem, rückfederungsfreiem Material, beispielsweise Silber. Solche Prothesen haben sich zwar bewährt, häufig reicht insbesondere auf die Dauer der Schließdruck nicht aus.

Bei Vorrichtungen der vorstehend beschriebenen Art, die auch unter der Bezeichnung "Sphinkter" bekannt sind, treten vielerlei Probleme auf. Insbesondere muß die jeweils gewünschte Schließ- bzw. Öffnungsfunktion einwandfrei und auf einfache und bequeme Weise erreichbar sein, ohne daß jedoch unzulässige Drücke oder andere Beanspruchungen des jeweiligen Organs und seiner Umgebung auftreten. Der Platzbedarf muß im Hinblick auf die Implantation im Körper klein bleiben. Schließlich soll die Implantation möglichst einfach erfolgen können, und der Herstellungsaufwand darf nicht zu hoch werden.

Der Erfindung liegt demgemäß die Aufgabe zugrunde, eine Vorrichtung zu schaffen, die die vorstehenden Anforderungen erfüllt. Zur Lösung der Aufgabe geht die Erfindung aus von einer Vorrichtung nach der vorgenannten DE-U-79 28 052 und ist dadurch gekennzeichnet, daß die Manschette elastisch ist und aus der Öffnungs- oder Schließstellung als Ruhestellung in die Schließ- bzw. Öffnungsstellung unter Auftreten einer elastischen Rüchstellkraft verformbar ist.

Eine bevorzugte Anwendung ist zwar eine Harninkentinenzprothese, aber die Vorrichtung kenn auch an praktisch allen anderen rohrförmigen Organen verwendet werden, beispielsweise am Harnleiter zur Verhinderung eines Rückflusses von Urin aus der Blase in Richtung zur Niere. Bei ausgeschalteten Abschnitten des Dünn- oder Dickdarms kann der jeweilige Darmabschnitt zum Reservoir werden, der mit Hilfe eines solchen Sphinkters kontinent wird. Die Ausleitung kann dabei zur Haut oder auch in Form einer Verbindung zu anderen körpereigenen Organen, z.B. zur Blase, zur Vagina, zu Hohlräumen, die durch plastische Operationen gebildet sind, oder auch zu anderen Darmabschnitten erfolgen. Ein Einsatz ist weiterhin im Bereich der Speiseröhre oder in Verbindung mit Magenabschnitten sowie als Verschlußmechanismus bei körpereigenen oder auch fremden Blasenersatzorganen möglich. Darüberhinaus kann der Sphinkter nach der vorliegenden Erfindung zur Erzielung einer Stuhlkontinenz und schließlich auch zur Einengung oder zum Verschließen von bestimmten Gefäßabschnitten Verwendung finden, um eine zeitweilige Unterbindung oder Verminderung der Blutdurchströmung zu erreichen.

Für die verschiedenen Einsatzzwecke sind Anpassungen der Abmessungen, der äußeren Form, der Spaltweite und der jeweiligen Rückstellkräfte erforderlich, die der Fachmann vornehmen kann. Sonderfälle hierzu werden noch erläutert. Der in der spaltförmigen Durchgangsöffnung auf das Organ ausgeübte Druck, der das Verschließen oder teilweise Verschließen bewirkt, läßt sich durch die Bemessung der Spaltweite sowie der Formsteifigkeit der Manschette und die Öffnungsgestaltung beeinflussen. Unter Formsteifigkeit soll dabei die Kraft verstanden werden, die zur Formveränderung der Manschette nötig ist, oder auch die elastische Rückstellkraft, die nach einer Verformung auftritt.

Unter "spaltförmige Durchgangsöffnung" bzw. "Spalt" wird hier zwar insbesondere ein gerader Spalt, aber auch ein gebogener oder mehrfach gekrümmter oder ein ringförmiger Spalt verstanden. Im weiteren Sinn soll "Spalt" darüber hinaus jede Durchgangsöffnung bezeichnen, deren Lumen sich durch äußere Kräfte beeinflussen läßt.

Die Vorrichtung nach der Erfindung kann so ausgeführt sein, daß sie in Ruhestellung das durch die Öffnung geführte Organ verschließt oder teilverschließt und zum Öffnen eine Vergrößerung der Spaltweite nötig ist, und zwar insbesondere durch eine Verformung der Manschette. Die Ausführung kann aber auch so getroffen sein, daß die Öffnungsstellung die Ruhestellung ist und zum Verschließen oder Teilverschließen des Organs die Spaltweite verengt wird.

Weiterbildungen der Erfindung sind Gegenstand der Unteransprüche. So kann die Manschette aus zwei über ein Scharnier verbundenen Halbschalen bestehen, deren freie Enden nach dem Anbringen an dem Organ verbindbar sind. Zweckmäßig wird das Scharnier durch eine Dünnstelle der Manschette gebildet, beispielsweise in Form eines Filmscharniers. Es ist zwar in manchen Fällen möglich, eine geschlossene Manschette über das jeweils zu verschliessende Organ zu schieben, in vielen Fällen, beispielsweise im Fall der Harnröhre, müßte das Organ jedoch dann zum Aufschieben der Manschette durchtrennt werden, was zu Komplikationen führen kann. Daher ist meist eine geöffnete Manschette entsprechend der vorstehenden Weiterbildung zweckmäßig. Nach dem Aufschieben der Manschette können die freien Enden miteinander vernäht werden, wozu zweckmäßig die freien Enden durch Lappen gebildet sind. Die Verbindung kann in einfacher Weise entsprechend einer weiteren Empfehlung der Erfindung aber auch mittels einer Verbindungsklammer erfolgen oder eines elatischen Rings, wenn die freien Enden auf ihrer Außenseite je eine Einlagerungsrille für die Klammer bzw. den Ring besitzen. Eine andere Möglichkeit besteht darin, daß an den freien Enden der Manschette ein Magnetverschluß angeordnet ist. Es kann dazu in jedes freie Ende ein Permanentmagnet in Form einer Leiste oder auch eines Ringes eingebracht sein, wobei die Polung so getroffen ist, daß sich die Magnete anziehen. Der eine Permanentmagnet läßt sich auch durch ein ferromagnetisches Material ersetzen. Die maximale Haltekraft wird außer von den magnetischen Eigenschaften dadurch bestimmt, welcher Luftspalt im geschlossenen Zustand verbleibt. Wenn die beiden Magnete direkt aneinanderliegen, ist die Haltekraft am größten. Dann muß aber ein Magnetmaterial verwendet werden, das implantierbar ist. Wenn die Permanentmagnete in die Manschette eingebettet sind, verbleibt wenigstens eine dünne Haut im "Luftspalt", über deren Dicke sich die Haltekraft einstellen läßt. Zusätzlich zu den Permanentmagneten kann eine Führung vorgesehen sein, die seitliche Verschiebungen der beiden freien Manschettenenden, insbesondere in geöffnetem Zustand, verhindert. Zur Verwirklichung der Führung läßt sich beispielsweise ein Stift an einem freien Ende anbringen, der in eine entsprechende Bohrung des anderen Endes eingreift. Schließlich kann vorgesehen sein, daß die beiden freien Enden einschließlich der Magnete durch einen Sack oder Beutel aus dünnem Kunststoffmaterial umhüllt sind. Dieser Sack verhindert, daß Körpergewebe zwischen die Verschlußteile einsprießt oder einwächst. Da der Sack erst nach dem Einlegen des Körperorgans angebracht werden kann, muß er entsprechende Schlitze aufweisen. Statt des Magnetverschlusses läßt sich an den freien Enden der Manschette auch ein Druckknopfverschluß anordnen, der ebenfalls wieder durch einen Sack oder Beutel umhüllbar ist. Eine andere Art der Verbindung, die zusätzlich eine Einstellung der Spaltweite ermöglicht, besteht darin, daß das eine freie Ende der Manschette eine bandartige Verlängerung mit Rastnocken oder -wülsten und das andere freie Ende einen an die Form der bandartigen Verlängerung angepaßten Durchgang aufweist. Die Verlängerung wird dann durch den Durchgang gesteckt und unter jeweiligem Einrasten nach jedem Nocken oder jedem Wulst soweit angezogen, bis die gewünschte Spaltweite erreicht ist.

Für die Ausbildung der Manschette selbst bestehen zahlreiche Möglichkeiten. So sieht eine Weiterbildung der Erfindung vor, daß die Manschette kreisförmigen oder ovalen Querschnitt besitzt und auf der Innenseite mit Gel gefüllte Kissen aufweist, die zwischen sich die spaltförmige Durchgangsöffnung definieren. Durch die gelgefüllten Kissen ist eine gleichmäßige Druckverteilung mit Polsterwirkung leicht erreichbar. Nach einem anderen Beispiel kann die Manschette im wesentlichen rechteckigen Querschnitt mit zwei sich gegenüberliegenden Wänden besitzen, die zwischen sich die spaltförmige Durchgangsöffnung definieren. Da keine zusätzlichen Gelkissen Verwendung finden, wird die in vielen Anwendungsfällen nachteilige Gefahr einer unterschiedlichen Alterung der Manschetten und der Kissen vermieden, wodurch sich Formänderungen ergeben könnten. Die Betätigung des Verschlusses kann dadurch vereinfacht werden, daß die parallel zur Durchgangsöffnung verlaufenden Schmalseiten der Manschette Griffmulden besitzen.

Zur Anpassung an die Form des durch die Öffnung geführten Organs oder an die Form, die dieses Organ nach dem Verschließen durch Aufweitung annimmt, kann mit Vorteil vorgesehen sein, daß die Durchgangsöffnung in der Durchflußrichtung eingangsseitig in Form eines Trichters aufgeweitet ist. Die Abdichtung ohne Ausübung eines zu großen Druckes läßt sich verbessern, wenn in Weiterbildung der Erfindung eine Begrenzungswand der Durchgangsöffnung wenigstens eine quer zur Durchgangsöffnung verlaufende Wulst aufweist. Zusätzlich kann in der anderen Begrenzungswand eine der Wulst gegenüberliegende Rinne gebildet sein.

Zur Festlegung des Verschlusses an der jeweiligen Implantationsstelle kann vorgesehen sein, daß an der Manschette Kunststoffäden oder ein Gewebe- oder Filzstreifen oder -stück aus Kunststoffäden angeordnet sind. Dabei kann die Anordnung durch Einbetten auf wenigstens einer Seite der Durchgangsöffnung und/oder an den Schmalseiten der Manschette erfolgen. Aus den Fäden können auch Ösen gebildet sein.

Häufig ist es erforderlich oder jedenfalls vorteilhaft, wenn ein Verschluß nach der Erfindung nicht nur eine Dauerschließstellung besitzt, die kurzzeitig durch Ausüben eines Druckes in eine Öffnungsstellung veränder-bar ist, derart, daß beispielsweise eine Harnentleerung erfolgen kann, sondern zusätzlich eine Daueröffnungsstellung einstellbar ist. Diese läßt sich beispielsweise nach der operativen Implantation verwenden, um bis zum Einheilen einen freien Harnablauf zu ermöglichen. Darüberhinaus kann zur Schonung des jeweils verschlossenen Gefäßes beispielsweise über Nacht die Daueröffnungsstellung gewählt werden, weil dann gegebenenfalls die Inkontinenz auch auf andere Weise versorgt werden kann. Zur Erzielung einer solchen Möglichkeit sieht eine Weiterbildung der Erfindung vor, daß wenigstens an einer Schmalseite der spaltförmigen Durchgangsöffnung ein nach außen abgeschlossener Raum mit wenigstens einem eingeschlossenen, von außen verschiebbaren Körper, vorzugsweise eine Kugel, vorgesehen ist. Der Raum kann dabei zweckmäßig in Richtung auf die Durchgangsöffnung einen Sitz aufweisen, der eine Kugel rastend aufnimmt, derart, daß die Daueröffnungsstellung erzielt ist. Durch das Verschieben des Körpers und vorzugsweise der Kugel wird nämlich eine Aufweitung des Spaltes erreicht, die die Öffnung bewirkt. Die Ausführung kann dabei so getroffen sein, daß eine leichte Druckausübung auf die Manschette eine kurzzeitige Öffnung ermöglicht und erst bei Ausübung eines stärkeren Druckes der Körper, so und gegebenenfalls in einen Sitz verschoben wird, daß die Öffnungsstellung permanent bleibt. Später läßt sich durch Rückverschiebung des Körpers unter manueller Druckausübung auf den Sphinkter wieder der Dauerschließzustand einstellen. Für die Ausbildung des Raumes oder Kugelraumes und seine Lage bestehen mehrere Möglichkeiten. So kann der Kugelraum etwa in der Spaltebene und quer zur Durchflußrichtung im rohrförmigen Körperorgan oder auch parallel zur Durchflußrichtung verlaufen. Der parallel zur Durchflußrichtung verlaufende Kugelraum kann an beiden Enden je einen Sitz für je eine Kugel aufweisen. Wenn die beiden Kugeln in ihrem Sitz liegen, ist das Körperorgan verschlossen. Drückt man mit vorzugsweise zwei Fingern einer Hand auf die beiden Kugeln und bringt sie aus ihrem Sitz in den zwischenliegenden Teil des Kugelraumes, so öffnet man die Manschette. Um die Verschiebung des Körpers bzw. der Kugel zu erleichtern, sieht eine Weiterbildung vor, daß ein Gleitmittel in den Kugelraum eingebracht ist. Hierzu eignet sich beispielsweise ein Gel oder auch ein Puder.

Je nach der Verwendung des Verschlusses läßt sich eine Implantation so vornehmen, daß die Bedienung von außen durch die Haut möglich ist. Es gibt jedoch auch Fälle, wo der Verschluß nicht auf einfache Weise tast- und bedienbar ist, beispielsweise bei einem Harnröhrenverschluß am Blasenausgang. Hierzu sieht die Erfindung in ihrer weiteren Ausbildung vor, daß der verschiebbare Körper, insbesondere eine Kugel, mit einer Fernbedienung nach Art eines Fernauslösers für eine fotografische Kamera versehen ist. Das Betätigungsorgan der Ferubedienung kann dann für den erläuterten Fall einer Anbringung des Verschlusses am Blasenausgang beispielsweise im Hodensack bzw. in den großen Schamlippen untergebracht sein. Die Fernbedienung weist zweckmäßig einen in Siliconkautschuk eingebetteten steifen Kunststoffschlauch auf, in dem ein von außen verschiebbares, auf den Körper bzw. die Kugel einwirkendes Betätigungsorgan gleitend gelagert ist. Das Betätigungsorgan kann ein Metalldraht, ein Seil, ein monophiler Kunststoffdraht oder auch eine drucksteife Wendel sein.

Eine andere Ausführungsform einer Vorrichtung nach der vorliegenden Erfindung besteht darin, daß wenigstens in einer Wand der Durchgangsöffnung ein gegen die Durchgangsöffnung durch eine dünne Haut abgeschlossener Raum vorgesehen ist, in dem ein Körper, insbesondere ein Stift oder eine Kugel, unter Änderung der Spaltweite der Durchgangsöffnung verschiebbar ist. Beim Verschieben wird durch eine entsprechende Ausbildung des durch die dünne Haut abgeschlossenen Raums, insbesondere eine Keilform, erreicht, daß die Haut beim Verschieben des Körpers mehr bzw. weniger stark ausgewölbt wird und dadurch die Spaltweite unter Verschließen bzw. Verengen bzw. Öffnen des durchgeführten Organs ändert. Unter "dünne Haut" wird dabei ein Gebilde verstanden, dessen Formsteifigkeit wesentlich geringer als die zugehörige Wand der Manschette ist, so daß sich beim Verschieben des Körpers bzw. der Kugel oder des Stiftes die Haut auswölbt und nicht die Manschette. Im Gegensatz zu den vorher beschriebenen Ausführungsbeispielen ergibt sich hier eine Daueröffenstellung, die erst durch das Verschieben des Körpers in eine Verschlußstellung oder Einengungsstellung verändert wird. Der Raum in einer Wand der Durchgangsöffnung kann mit Vorteil eine oder mehrere Raststellen für den Körper besitzen, so daß sich definierte Stellungen bequem einstellen lassen. Die oben beschriebene Fernbedienung kann bei diesem Ausführungsbeispiel ebenfalls mit Vorteil benutzt werden.

Zur Erzielung der Öffnungs- bzw. Schließstellung oder auch einer Daueröffnungs- bzw. Dauerschließstellung läßt sich in Weiterbildung der Erfindung statt des nach außen abgeschlossenen Raumes mit einem von außen verschiebbaren Körper auch an wenigstens einer Schmalseite der spaltförmigen Durchgangsöffnung ein geschlossener Druckraum vorsehen, der mit einer Pumpvorrichtung in Verbindung steht, wobei der Druckraum und die Pumpvorrichtung mit einer Druckflüssigkeit gefüllt sind. Die Pumpvorrichtung kann nahe an der Manschette angeordnet und beispielsweise einstückig mit ihr ausgebildet sein, bei einer Verbindung über einen Schlauch läßt sich aber auch eine Fernbedienung verwirklichen. Die Pumpvorrichtung kann im einfachsten Fall aus einem Druckkissen bestehen, es läßt sich aber auch eine Kolbenpumpe mit einem verschiebbaren Kolben verwenden. Die Daueröffnungs- bzw. Dauerschließstellung kann beispielsweise dadurch erzielt werden, daß im Zuge der Verbindung zwischen der Druckkammer und der Pumpvorrichtuug ein Ventil vorgesehen ist, das einen Rückfluß verhindert.

Als Material für den verschiebbaren Körper, insbesondere eine Kugel, kann bei allen Ausführungsbeispielen Polytetrafluoräthylen (Teflon) benutzt werden. Eine Kugel bzw. ein Körper aus diesem Material ist leicht und glatt und wird auch bei Eindiffundieren von Flüssigkeiten nach der Implantation nicht beeinträchtigt. Die Manschette wird zweckmäßig aus Siliconkautschuk mit einer für medizinische zwecke zugelassenen Qualität hergestellt. Dabei können unterschiedliche Härtegrade für die einzelnen Teile der Vorrichtungen gewählt werden. Es können aber auch andere Materialien Verwendung finden, beispielsweise Kugeln aus rostfreiem Stahl oder Manschetten aus anderen Kuststoffen, beispielsweise Polyurethan.

Nachfolgend werden Ausführungsbeispiele der Erfindung anhand der Zeichnungen beschrieben. Es zeigen:

Fig. 1 und 2 die Querschnitts- bzw. Seitenansicht eines Ausführungsbeispiels der Erfindung mit Kreisquerschnitt und Gelkissen;

Fig. 3 und 4 ein weiteres Ausführungsbeispiel der Erfindung mit ovalem Querschnitt und Gelkissen;

Fig. 5 eine Verschlußklammer für das Ausführungsbeispiel nach Fig. 3 und 4;

Fig. 6 und 7 die Seitenansicht bzw. den Querschnitt eines Ausführungsbeispiels der Erfindung mit rechteckigem Querschnitt ohne Verwendung von Gelkissen;

Fig. 8 einen vergrößerten Längsschnitt durch das Ausführungsbeispiel gemäß Fig. 6 und 7;

Fig. 9 ein Ausführungsbeispiel ähnlich Fig. 6 bis 8, wobei jedoch eine Daueröffnungsstellung einstellbar ist;

Fig. 10 eine Abwandlung des Ausführungsbeispiels nach Fig. 9;

Fig. 11 und 12 ein Ausführungsbeispiel ähnlich Fig. 9 mit Öffnung des Verschlusses durch Druckausübung bzw. Daueröffnung mittels einer Kugel;

Fig. 13 ein Ausführungsbeispiel ähnlich Fig. 9, wobei jedoch Kugeln zur Erzielung einer Daueröffnung auf beiden Seiten der Manschette vorgesehen sind;

Fig. 14 schematisch die Anbringungsmöglichkeiten für einen Harnröhrensphinkter beim Mann;

Fig. 15 ein Ausführungsbeispiel ähnlich Fig. 9 mit zusätzlicher Fernbedienung;

Fig. 16 eine Abwandlung des Ausführungsbeispiels nach Fig. 15;

Fig. 17 eine weitere Abwandlung des Ausführungsbeispiels nach Fig. 15;

Fig. 18 das Betätigungsorgan einer Fernbedienung für die Ausführungsbeispiele nach Fig. 15 bis 18;

Fig. 19 ein weiteres Ausführungsbeispiel der Erfindung;

Fig. 20 eine Abwandlung des Ausführungsbeispiels nach Fig. 6 bis 8 mit einstellbarer Spaltweite;

Fig. 21 ein Ausführungsbeispiel der Erfindung mit einem Magnetverschluß;

Fig. 22 eine Abwandlung des Ausführungsbeispiels nach Fig. 10 mit zwei verschiebbaren Kugeln;

Fig. 23 ein Ausführungsbeispiel der Erfindung mit hydraulischer Betätigung.

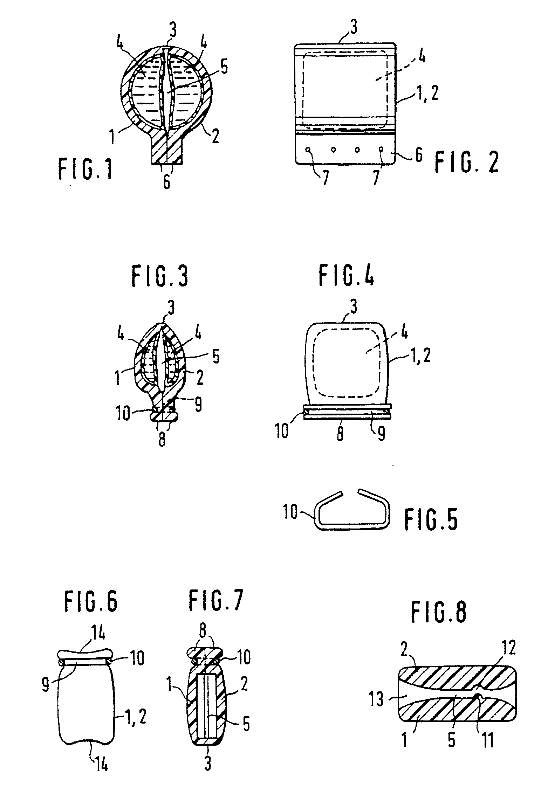

Das Ausführungsbeispiel gemäß Fig. 1 und 2 weist eine geteilte Manschette mit zwei Halbschalen 1,2 aus Siliconkautschuk auf, die über ein nur schematisch erkennbares Filmscharnier 3 verbunden sind. Im Innern der Halbschalen ist je ein mit einem Gel gefülltes Kissen 4 angeordnet, zwischen denen sich eine spaltförmige Durchgangsöffnung 5 für das jeweilige zu verschließende Organ, insbesondere die Harnröhre beim Menschen, befindet. Auf der dem Filmscharnier 3 abgewandten Seite weisen die Halbschalen 1,2 angeformte Lappen 6 auf, die nach dem Anbringen beispielsweise auf der Harnröhre eine dauerhafte Verbindung durch Nähen ermöglichen. Zur Erleichterung sind hierzu vorgefertigte Bohrungen 7 angebracht. Die Spaltweite der Durchgangsöffnung 5 ist so gewählt, daß in der gezeigten Ruhestellung gemäß Fig. 1 eine durch die Öffnung führende Harnröhre (nicht dargestellt) durch Druckausübung auf deren Wände verschlossen ist. Die Einstellung kann dabei so erfolgen, daß der Verschluß bis zu einem bestimmten Druck erhalten bleibt, beispielsweise bis zu einem Druck von 70 cm Wassersäule, so daß ein Notabfluß oberhalb dieses Druckes möglich ist. Die Öffnungsstellung wird erreicht, indem ein seitlicher Druck mit den Fingern zwischen dem Filmscharnier 3 und den Lappen 6 ausgeübt wird. Dann erweitert sich die spaltförmige Durchgangsöffnung 5 und gibt den Durchfluß durch die Harnröhre frei. Bei stärkerem Druck erfolgt dann wieder ein Verschließen, weil die Durchgangsöffnung 5 sich dann in der Querrichtung verengt. Das kann in manchen Anwendungsfällen zweckmäßig sein.

Das Ausführungsbeispiel gemäß Fig. 3 und 4 weicht von dem Ausführungsbeispiel gemäß Fig. 1 und 2 durch die ovale Querschnittsform der Halbschalen 1,2 und die Art des Verschlusses ab. Die freien Enden der Halbschalen 1,2 besitzen hinter wulstförmigen Verdickungen 8 eine Einlagerungsrille 9, in die eine in Fig. 5 gezeigte Klammer 10 aus beispielsweise rostfreiem Stahl eingesetzt und durch Zubiegen befestigt werden kann. Wegen der ovalen Form des Ausführungsbeispiels nach Fig. 3 und 4 ist eine stärkere Aufweitung der Durchgangsöffnung 5 möglich, beispielsweise zum Durchschieben eines Katheders oder eines Endoskops oder auch zum Entleeren.

Das Ausführungsbeispiel gemäß Fig. 6 bis 8 besitzt abweichend von den zuvor beschriebenen Ausführungsbeispielen keine Gelkissen 4. Die spaltförmige Durchgangsöffnung 5 befindet sich hier direkt zwischen den Halbschalen 1, 2. Wie die vergrößerte Querschnittsdarstellung gemäß Fig. 8 erkennen läßt, ist zusätzlich in der Durchgangsöffnung zur Verbesserung der Abdichtwirkung eine Querwulst 11 in der einen Halbschale 1 und gegenüber eine angepaßte Rille 12 in der anderen Halbschale 2 vorgesehen. Die Wulst 11 und die Rille 12 sind in der Durchgangsöffnung 5 nicht in der Mitte, sondern etwas gegen den Ausgang versetzt angeordnet, wobei die Durchflußrichtung in der Darstellung gemäß Fig. 8 von links nach rechts verläuft. Eingangsseitig (mit Bezug auf die Durchflußrichtung) besitzt die spaltförmige Öffnung 5 eine trichterförmige Ausweitung 13, die eine Anpassung an die Form einer durch die Öffnung 5 verlaufenden Harnröhre (nicht gezeigt) darstellt, wenn man berücksichtigt, daß nach dem Verschließen der Harnröhre sich eine Aufweitung einstellt. Das Öffnen des Sphinkters gemäß Fig. 6 bis 8 erfolgt wiederum durch Druckausüben auf die Schmalseiten, wobei die Druckausübung durch Griffmulden 14 erleichtert ist. Außerdem ergibt sich durch die trichterförmige Ausbildung 13 eine kurzzeitige Verbesserung der Abdichtung. Wenn nämlich beispielsweise beim Husten der Trichter 13 etwas aufgeweitet wird, kommen die Wände der Halbschalen 1,2 auf der Ausgangsseite näher zusammen, so daß die Spaltweite kleiner wird.

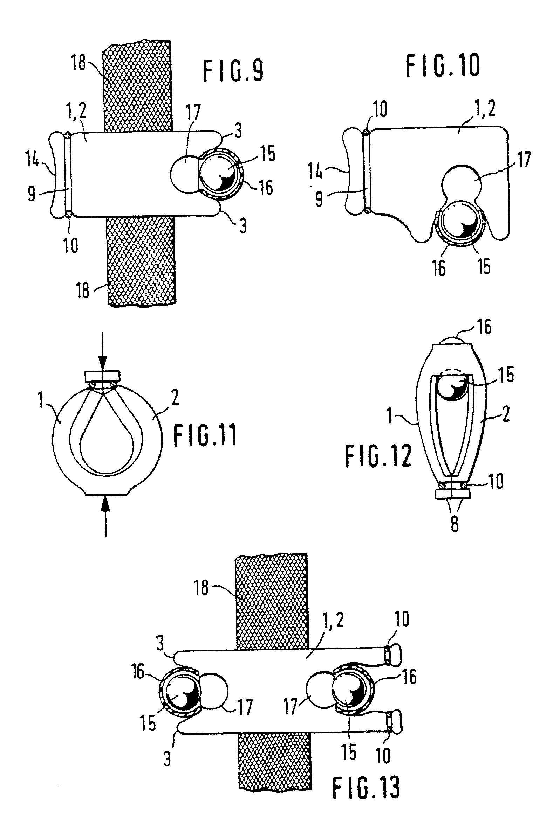

Das Ausführungsbeispiel gemäß Fig. 9 ermöglicht zusätzlich neben der Schließstellung und der kurzzeitigen Öffnungsstellung durch Druckausübung eine Daueröffnungsstelle mit Hilfe einer Kunststoffkugel 15, die in einem nach außen durch einen Sack 16 abgeschlossenen und dichten Raum im Bereich zwischen dem geteilten Filmscharnier 13 angeordnet ist. Unter Druck von außen läßt sich die Kugel 16 in der Darstellung nach links in einen Sitz 17 verschieben, wobei die spaltförmige Durchgangsöffnung 5 aufgeweitet wird und damit eine Daueröffnungsstellung erzielt ist. Diese Stellung ist in Fig. 12 dargestellt. Fig. 11 soll zusätzlich veranschaulichen, daß sich eine große Öffnung auch ohne Kugel durch einfache Druckausübung erzielen läßt.

Bei dem Ausführungsbeispiel gemäß Fig. 9 verläuft der Kugelraum hinsichtlich der Verschiebungsrichtung für die Kugel 15 etwa in der Spaltebene, also in der Ebene der spaltförmigen Durchgangsöffnung 5 und gleichzeitig quer zur Durchflußrichtung, die in der Darstellung gemäß Fig. 9 von oben nach unten führt. Das Ausführungsbeispiel gemäß Fig. 10 ist dahingehend abgewandelt, daß der Kugelraum zwar ebenfalls in der Spaltebene, nunmehr aber parallel zur Durchflußrichtuug verläuft, die in der Darstellung nach Fig. 10 von oben nach unten führt.

Das Ausführungsbeispiel gemäß Fig. 13 entspricht dem Ausführungsbeispiel gemäß Fig. 9, besitzt aber zusätzlich auch auf der Verschlußseite der beiden Halbschalen 1,2 einen abgeschlossenen Kugelraum mit einer Kunststoffkugel 15. Dieser Raum kann dann nicht zwischen den beiden Halbschalen 1,2 untergebracht sein (vgl. Fig. 12), sondern muß wegen der Abdichtung in einer der Halbschalen 1 oder 2 liegen. Durch Eindrücken beider Kugeln 15 in ihren Sitz 17 kann eine symmetrische und große Durchgangsöffnung mit Daueröffnungsstellung erzielt werden. Abweichend vom Ausführungsbeispiel nach Fig. 9 werden zwei Verschlußklammern 10 verwendet.

Bei allen Ausführungsbeispielen mit einer Daueröffnungsstellung unter Verwendung einer verschiebbaren Kugel ist der jeweilige Kugelraum zur Verbesserung der Gleiteigenschaften mit einem Gel (nicht dargestellt) gefüllt. Bei den Ausführungsbeispielen gemäß Fig. 9 und 13 ist außerdem gezeigt, daß in eine der Halbschalen 1,2 oder auch in beide Halbschalen ein Gewebestreifen 18 aus Kunststoffäden einvulkanisiert werden kann. Dieser Gewebestreifen vereinfacht die Festlegung des Sphinkters an der jeweiligen Implantationsstelle.

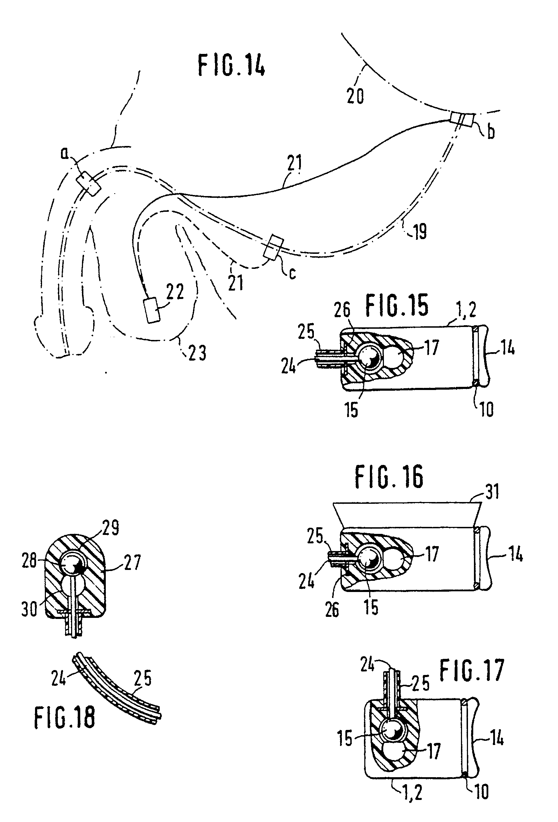

In Fig. 14 ist schematisch dargestellt, an welchen Stellen der Harnröhre 19 ein Verschluß nach einem der Ausführungsbeispiele der Erfindung beim Mann angebracht werden kann. Wenn der Verschluß gemäß a im Bereich des Penis angeordnet wird, ist eine direkte Betätigung durch Druckausübung mit den Fingern möglich. Es können demgemäß die Ausführungsformen entsprechend Fig. 1 bis 13 Verwendung finden. Wenn dagegen der Verschluß entsprechend b am Ausgang der Blase 20 oder entsprechend c unterhalb des Beckenbodens (nicht gezeigt) angeordnet wird, läßt sich der Verschluß nicht ohne weiteres direkt betätigen. In diesem Fall wird eine Fernbedienung 21 benutzt, die ähnlich wie ein Fernauslöser für eine fotografische Kamera ausgebildet ist. Das Betätigungsorgan 22 ist dann beispielsweise im Hodensack 23 untergebracht. In entsprechender Weise kann das Betätigungsorgan für einen fernbedienbaren Verschluß am Blasenausgang der Frau in den großen Schamlippen untergebracht sein.

Fig. 15 zeigt eine erste Ausführungsform eines Blasensphinkters mit Fernbedienung. Das Ausführungsbeispiel entspricht dem Ausführungsbeispiel nach Fig. 9, wobei jedoch die Kunststoffkugel 15 nicht durch direkte Druckausübung verschoben wird, sondern mittels eines Stehldrahtes oder einer Wendel 24, die in einem Schlauch 25 aus festem Kunststoff läuft. Die Verankerung 26 des Kunststoffschlauches ist schematisch in Form einer Platte 26 dargestellt.

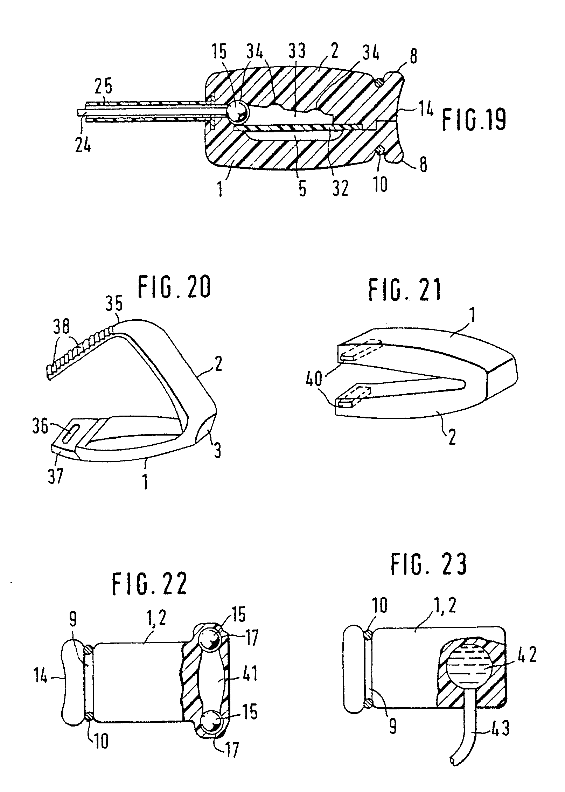

Fig. 18 zeigt das zugeordnete Betätigungsorgan 27, zu dem der Schlauch 25 mit der inneren Wendel 24 hinführt. Das Betätigungsorgan 27, das ebenfalls aus Siliconkautschuk hergestellt ist, enthält in einem abgeschlossenen Raum eine Kunststoffkugel 28, die an der Wendel 24 befestigt ist und zwischen zwei Sitzen 29,30 verschiebbar ist. In der in Fig. 15 und 18 dargestellten Stellung der Fernbedienung ist der Sphinkter geschlossen. Wenn die Kugel 28 und damit die Kugel 15 nur geringfügig durch Druckausübung verschoben wird, ohne jedoch vom Sitz 29 in den Sitz 30 zu gelangen, so findet eine Aufweitung der spaltförmigen Durchgangsöffnung so weit statt, daß eine ausreichende Öffnung für das Harnablassen erreicht wird. Anschließend kann durch Druckentlastung wieder in die Schließstellung gegangen werden. Erst wenn die Kugel 28 über eine Raststelle hinaus in den Sitz 30 gedrückt wird, bewegt sich auch die Kugel 15 in ihren anderen Sitz, so daß dann eine Daueröffnungsstellung erreicht ist, die ebenfalls und häufig bequemer für die Harnentleerung benutzt werden kann. Die vorstehend beschriebene kurzzeitige Öffnungsstellung kann natürlich auch bei den Ausführungsbeispielen nach Fig. 9,10,13 verwendet werden.

Das Ausführungsbeispiel gemäß Fig. 16 unterscheidet sich von dem Ausführungsbeispiel nach Fig. 15 nur durch die zusätzliche Anbringung eines trichterförmigen Ansatzes 31 auf der Eingangsseite der Durchgangsöffnung. Dieser Trichter 31 ermöglicht eine Anpassung an den Blasenhals entsprechend b in Fig. 14. Das Ausführungsbeispiel gemäß Fig. 15 kann beispielsweise an der Stelle c der Fig. 14 eingesetzt werden. Das Ausführungsbeispiel gemäß Fig. 17 zeigt eine Abwandlung des Ausführungsbeispiels nach Fig. 15, ähnlich wie die Abwandlung in Fig. 10, wobei hier der Raum für die Kugel 15 und demgemäß der Verschiebeweg der Wendel 24 parallel zur Durchflußrichtung (vertikal in Fig. 17) verläuft.

In Fig. 19 ist ein Ausführungsbeispiel dargestellt, das ebenfalls mit einer Fernbedienung betätigbar ist, im übrigen aber von den bisher beschriebenen Ausführungsbeispielen in einigen Punkten abweicht. Die Durchgangsöffnung 5 befindet sich nämlich hier zwischen der Wand der einen Halbschale 1 und einer verhältnismäßig dünnen Haut 32. Die gezeigte Lage entspricht der Öffnungsstellung, wenn man sich vorstellt, daß beispielsweise eine Harnröhre durch die Öffnung führt (senkrecht auf der Zeichnungsebene) und dabei durch ihre Eigenelastizität die Haut 32 gegebenenfalls geringfügig hochwölbt. Zur Erzielung eines teilweisen oder vollständigen Verschlusses muß die Kugel 15 dann mittels der Wendel 24 aus der gezeigten Lage in einem keilartig zulaufenden Raum 33 verschoben werden, wobei die Haut 32 zunehmend in Richtung auf das zu verschließende Organ gedrückt wird. Raststellen 34 für die Kugel 15 ermöglichen das definierte Einstellen bestimmter Durchflußzustände. Abweichend von den früheren Ausführungsbeispielen besitzt das Ausführungsbeispiel von Fig. 19 also eine Daueröffnungsstellung im Ruhezustand und kann aus dieser Stellung in die teilweise oder vollständige Schließstellung gebracht werden. Allerdings lassen sich auch die früheren Ausführungsbeispiele auf verhältnismäßig einfache Weise so umkonstruieren, daß beim Eindrücken der Kugeln eine Dauerschließstellung erreicht wird.

Das Ausführungsbeispiel gemäß Fig. 20 stellt eine Abwandlung des Ausführungsbeispiels gemäß Fig. 6 bis 8 dar. Anstelle der Verschlußmöglichkeit der beiden Halbschalen 1,2 mittels einer Klammer 10 ist im vorliegenden Fall die eine Halbschale 2 mit einer bandförmigen Verlängerung 35 versehen, die durch eine Öffnung 36 eines Lappens 37 an der anderen Halbschale 1 gesteckt werden kann. Auf der Verlängerung 35 sind Rastnocken in Form querverlaufender Wulste 38 mit etwa rechteckigem Querschnitt angeordnet, so daß das Band 35 bis zu einer gewünschten Stelle, d.h., bei einer bestimmten Spaltweite, in die Öffnung 36 eingeschoben werden kann und dort durch die Rastung festgelegt ist. Die bandartige Verlängerung 35 kann dabei zweckmäßig aus härter eingestelltem Material (Siliconkautschuk) wie die Halbschalen 1,2 bestehen. Ein ähnlicher Verschluß wie bei dem Ausführungsbeispiel nach Fig. 20 läßt sich auch bei den anderen Ausführungsbeispielen verwenden.

Bei einem weiteren Ausführungsbeispiel der Erfindung gemäß Fig. 21 sind die beiden Halbschalen 1,2 im dargestellten Ruhezustand gespreizt. Drückt man auf die beiden freien Enden der Halbschalen 1,2, so nähern sich zwei dort eingebettete Magnetstäbe 40 aus permanentmagnetischem Material, die so magnetisiert und gepolt sind, daß sie sich anziehen. Die Höhe der Haltekraft läßt sich durch die Stärke der Magneten und auch die Dicke des Kunststoffmaterials bestimmen, das beim Zusammendrücken der Halbschalen 1,2 zwischen den Magneten verbleibt. Die Oberfläche der Magneten kann auch aus dem Kunststoffmaterial herausragen, so daß denn eine sehr große Halte- oder Haftkraft erzielbar ist. Um zu vermeiden, daß insbesondere im geöffneten Zustand Gewebe in den Spalt zwischen den Magneten 40 einwächst, können die freien Enden der Halbschalen 1,2 mit einem Sack oder Beutel (nicht gezeigt) umhüllt sein.

Das Ausführungsbeispiel nach Fig. 22 stellt eine Abwandlung des Ausführungsbeispiels nach Fig. 10 dar. Auch hier verläuft der Kugelraum 41 parallel zur Durchflußrichtung, enthält aber zwei Kugeln 15. In der dargestellten Ruhelage, wenn also der Durchfluß gesperrt ist, liegen die Kugeln 15 je in einem Sitz 17 am Ende des Kugelraumes 41. Zur Erzielung einer Daueröffnungsstellung wird vorzugsweise mit den Fingern einer Hand Druck auf beide Kugeln 15 ausgeübt, die dann ihren Sitz 17 verlassen und unter Aufspreizen der beiden Halbschalen 1,2 etwa in die Mitte des Kugelraumes 41 gelangen. Durch einen Fingerdruck in der Querrichtung können die beiden Kugeln 15 wieder in ihren Sitz 17 zurückgebracht werden.

Ein zusätzliches Ausführungsbeispiel ist in Fig. 23 dargestellt. Abweichend von den bisher beschriebenen Ausführungsbeispielen erfolgt das Öffnen und Schließen der beiden Halbschalen 1,2 nicht durch manuelles Druckausüben und gegebenenfalls Verschieben einer Kugel, sondern es ist an einer Schmalseite ein geschlossener Druckraum 42 vorgesehen, der über einen Schlauch 43 mit einer Pumpvorrichtung (nicht gezeigt) in Verbindung steht. Der Druckraum 42, der Schlauch 43 und die Pumpvorrichtung sind mit einer Druckflüssigkeit gefüllt, so daß bei einer Betätigung der Pumpvorrichtung eine Aufweitung des Druckraumes 42 und damit ein Öffnen des Verschlusses erzielt werden kann. Das zum Öffnen erforderliche Volumen an Druckflüssigkeit ist nur sehr klein und beträgt beispielsweise etwa 1 ml. Die Pumpvorrichtung kann aus einer an der Manschette 1,2 angebrachten Druckkammer (nicht gezeigt) bestehen. Eine solche Druckkammer oder auch eine andere Pumpvorrichtung kann jedoch auch abgesetzt über einen längeren Schlauch 43 vorgesehen sein, so daß eine Fernbetätigung möglich wird. Wenn eine Daueröffnungsstellung erwünscht ist, muß entweder die Pumpvorrichtung entsprechend ausgebildet sein oder es kann ein einseitig wirkendes Ventil (nicht gezeigt) in dem Schlauch 43 angeordnet werden.

**Ansprüche**

1. Implantierbare Vorrichtung zum wahlweisen Öffnen und wenigstens teilweisen Verschließen von rohrförmigen Körperorganen, insbesondere der Harnröhre, mit einer elastomeren Kunststoff aufweisenden Manschette, die eine spaltförmige Durchgangsöffnung für das Organ besitzt, wobei die Weite der Durchgangsöffnung zur Erzielung der Öffnungs- bzw. Schließstellung durch Verformen der Manschette veränderbar ist, dadurch gekennzeichnet, daß die Manschette (1, 2) elastisch ist und aus der Öffnungs- oder Schließstellung als Ruhestellung in die Schließ- bzw. Öffnungsstellung unter Auftreten einer elastischen Rückstellkraft verformbar ist.

2. Vorrichtung nach Anspruch 1, dadurch gekennzeichnet, daß die Manschette aus zwei über ein Scharnier (3) verbundenen Halbschalen (1,2) besteht, deren freie Enden (6;8; 35,37) nach dem Anbringen an dem Organ verbindbar sind.

3. Vorrichtung nach Anspruch 2, dadurch gekennzeichnet, daß das Scharnier durch eine Dünnstelle (3) der Manschette (1,2), beispielsweise in Form eines Filmscharniers, gebildet ist.

4. Vorrichtung nach Anspruch 2 oder 3, dadurch gekennzeichnet, daß die freien Enden der Manschette (1,2) durch miteinander vernähbare Lappen (6) gebildet sind.

5. Vorrichtung nach Anspruch 2 oder 3, dadurch gekennzeichnet, daß die freien Enden auf ihrer Außenseite je eine Einlagerungsrille (9) für eine Verbindungsklammer (10) oder einen elastischen Ring aufweisen.

6. Vorrichtung nach Anspruch 2 oder 3, dadurch gekennzeichnet, daß an den freien Enden der Manschette (1,2) ein Magnetverschluß (40) angeordnet ist (Fig. 21).

7. Vorrichtung nach Anspruch 2 oder 3, dadurch gekennzeichnet, daß an den freien Enden der Manschette ein Druckknopfverschluß angeordnet ist.

8. Vorrichtung nach Anspruch 2 oder 3, dadurch gekennzeichnet, daß das eine freie Ende der Manschette (2) eine bandartige Verlängerung (35) mit Rastnocken oder -wülsten (38) und das andere freie Ende (37) einen an die Form der bandartigen Verlängerung angepaßten Durchgang (36) aufweist (Fig. 20).

9. Vorrichtung nach einem der Ansprüche 1 bis 8, dadurch gekennzeichnet, daß die Manschette (1,2) kreisringförmigen oder ovalen Querschnitt besitzt und auf der Innenseite mit gelgefüllten Kissen (4) ausgestattet ist, die zwischen sich die spaltförmige Durchgangsöffnung (5) definieren (Fig. 1 bis 4).

10. Vorrichtung nach einem der Ansprüche 1 bis 8, dadurch gekennzeichnet, daß die Manschette (1,2) im wesentlichen rechteckigen Querschnitt besitzt und zwei sich gegenüberliegende Wände aufweist, die zwischen sich die spaltförmige Durchgangsöffnung (5) definieren.

11. Vorrichtung nach Anspruch 10, dadurch gekennzeichnet, daß die parallel zur Durchgangsöffnung (5) verlaufenden Schmalseiten der Manschette Griffmulden (14) besitzen.

12. Vorrichtung nach einem der Ansprüche 1 bis 11, dadurch gekennzeichnet, daß die Durchgangsöffnung (5) in der Durchflußrichtung eingangsseitig in Form eines Trichters (13,31) aufgeweitet ist (Fig. 8,16).

13. Vorrichtung nach einem der Ansprüche 1 bis 12, dadurch gekennzeichnet, daß eine Begrenzungswand (11) der Durchgangsöffnung (5) wenigstens eine quer zur Durchgangsöffnung verlaufende Wulst (11) aufweist (Fig. 8).

14. Vorrichtung nach Anspruch 13, dadurch gekennzeichnet, daß in der anderen Begrenzungswand (2) eine der Wulst (11) gegenüberliegende Rinne (12) gebildet ist (Fig. 8).

15. Vorrichtung nach einem der Ansprüche 1 bis 14, dadurch gekennzeichnet, daß an der Manschette (1,2) Kunststoffäden oder eine Gewebe- oder Filzstreifen oder -stück (18) aus Kunststoffäden angeordnet sind (Fig. 9,13)

16. Vorrichtung nach Anspruch 15, dadurch gekennzeichnet, daß die Kunststoffäden oder ein Gewebe- oder Filzstreifen oder -stück (18) aus Kunststoffäden wenigstens auf einer Seite der Durchgangsöffnung in die Wand der Manschette (1,2) eingebettet sind (Fig. 9,13).

17. Vorrichtung nach Anspruch 15, dadurch gekennzeichnet, daß die Kunststoffäden oder ein Gewebe- oder Filzstreifen oder -stück aus Kunststoffäden an den Schmalseiten der Manschette in deren Wand eingebettet sind.

18. Vorrichtung nach einem der Ansprüche 1 bis 17, dadurch gekennzeichnet, daß wenigstens an einer Schmalseite der spaltförmigen Durchgangsöffnung (5) ein nach außen abgeschlossener Raum mit wenigstens einem eingeschlossenen, von außen verschiebbaren Körper, vorzugsweise eine Kugel (15), vorgesehen ist.

19. Vorrichtung nach Anspruch 18, dadurch gekennzeichnet, daß der Raum in Richtung auf die Durchgangsöffnung einen Sitz (17) aufweist, der eine Kugel (15) rastend aufnimmt, derart, daß eine Daueröffnungsstellung für das Organ erzielt ist.

20. Vorrichtung nach Anspruch 19, dadurch gekennzeichnet, daß der Kugelraum etwa in der Spaltebene und quer zur Durchflußrichtung in rohrförmigen Körperorgan verläuft (Fig. 9).

21. Vorrichtung nach Anspruch 19, dadurch gekennzeichnet, daß der Kugelraum etwa in der Spaltebene und parallel zur Durchflußrichtung im rohrförmigen Körperorgan verläuft (Fig. 10).

22. Vorrichtung nach Anspruch 21, dadurch gekennzeichnet, daß der Kugelraum (41) an beiden Enden je einen Sitz (17) für je eine Kugel (15) besitzt (Fig. 22).

23. Vorrichtung nach einem der Ansprüche 19 bis 22, dadurch gekennzeichnet, daß der Kugelraum ein Gleitmittel enthält.

24. Vorrichtung nach einem der Ansprüche 1 bis 17, dadurch gekennzeichnet, daß wenigstens in einer Wand (1) der Durchgangsöffnung (5) ein gegen die Durchgangsöffnung durch eine dünne Haut (32) abgeschlossener Raum (33) vorgesehen ist, in dem ein Körper, insbesondere ein Stift oder eine Kugel (15),unter Änderung der Spaltweite der Durchgangsöffnung (5) verschiebbar ist (Fig. 19).

25. Vorrichtung nach Anspruch 24, dadurch gekennzeichnet, daß der Raum (33) wenigstens eine Raststelle (34) für den Körper (15) besitzt.

26. Vorrichtung nach einem der Ansprüche 18 bis 25, dadurch gekennzeichnet, daß der verschiebbare Körper (15) mit einer Fernbedienung (24 bis 30) nach Art eines Fernauslösers für eine fotografische Kamera versehen ist (Fig. 15 bis 19).

27. Vorrichtung nach Anspruch 26, dadurch gekennzeichnet, daß die Fernbedienung einen in Siliconkautschuk eingebetteten steifen Kunststoffschlauch (25) aufweist, in dem ein von außen verschiebbares, auf den Körper (15) einwirkendes Betätigungsorgan (24) gleitend gelagert ist.

28. Vorrichtung nach einem der Ansprüche 18 bis 27, dadurch gekennzeichnet, daß der verschiebbare Körper, insbesondere eine Kugel (15), aus Polytetrafluoräthylen besteht.

29. Vorrichtung nach einem der Ansprüche 1 bis 17, dadurch gekennzeichnet, daß wenigstens an einer Schmalseite der spaltförmigen Durchgangsöffnung (5) ein geschlossener Druckraum (42) angeordnet ist, der mit einer Pumpvorrichtung in Verbindung steht, und daß der Druckraum (42) und die Pumpvorrichtung mit einer Druckflüssigkeit gefüllt sind (Fig.23).

30. Vorrichtung nach Anspruch 29, dadurch gekennzeichnet, daß der Druckraum (42) über einen Schlauch (43) mit der Pumpvorrichtung in Verbindung steht.

31. Vorrichtung nach einem der vorhergehenden Ansprüche, dadurch gekennzeichnet, daß die Manschette (1,2) aus Siliconkautschuk besteht.

## Claims

1. An implantable device for selectively opening and at least partly closing tubular body organs, more particularly the urethra, the device comprising a sleeve made or elastomeric plastics and formed with a slit-like through opening for the organ, the width of the opening being variable so as to bring about the open or closed position by deforming the sleeve, characterised in that the sleeve (1, 2) is resilient and can be deformed from the open or closed position (the inoperative position) into the closed or open position, accompanied by a resilient restoring force.

2. A device according to claim 1, characterised in that the sleeve is made up of two half-shells (1, 2) connected by a hinge (3) and having free ends (6; 8; 35, 37) which can be connected after attaching to the organ.

3. A device according to claim 2, characterised in that the hinge is formed by a thin place (3) in the sleeve (1, 2) and is e.g. a film hinge.

4. A device according to claim 2 or 3, characterised in that the free ends of the sleeve (1, 2) are formed by tabs (6) which can be sewn together.

5. A device according to claim 2 or 3, characterised in that on the outside, the free ends each have a groove (9) for receiving a connecting clamp (10) or a resilient ring.

6. A device according to claim 2 or 3, characterised in that a magnetic closure means (40) is disposed on the free ends of the sleeve (1, 2) (Fig. 21).

7. A device according to claim 2 or 3, characterised in that a press-button closure means is disposed on the free ends of the sleeve.

8. A device according to claim 2 or 3, characterised in that one free end of the sleeve (2) has a strip-like prolongation (35) formed with locking cams or beads (38) and the other free end (37) is formed with an aperture (38) adapted to the shape of the strip-like prolongation (Fig. 20).

9. A device according to any of claims 1 to 8, characterised in that the sleeve (1, 2) has a circular or oval cross-section and on its inside is provided with gel-filled pads (4) which define the slit-like through opening (5) between them (Figs. 1 to 4).

10. A device according to any of claims 1 to 8, characterised in that the sleeve (1, 2) has a substantially rectangular cross-section and two opposite walls which between them define the slit-like through opening (5).

11. A device according to claim 10, characterised in that the narrow sides of the sleeve extending parallel to the through opening (5) have recesses (14) for gripping.

12. A device according to any of claims 1 to 11, characterised in that in the flow direction, the through opening (5) is widened in the form of a funnel (13, 31) at the entry end (Figs. 8 and 18).

13. A device according to any of claims 1 to 12, characterised in that a boundary wall (11) of the through opening (5) comprises at least one bead (11) extending transversely to the through opening (Fig. 8).

14. A device according to claim 13, characterised in that a channel (12) opposite the bead (11) is formed in the other boundary wall (2) (Fig. 8).

15. A device according to any of claims 1 to 14, characterised in that plastics threads or a fabric or felt strip or piece (18) made of plastics threads are disposed on the sleeve (1, 2) (Figs. 9 and 13).

16. A device according to claim 15, characterised in that the plastics threads or a fabric or felt strip or piece (18) made of plastics threads are embedded in the wall of the sleeve (1, 2) on one side of the through opening (Figs. 8 and 13).

17. A device according to claim 15, characterised in that the plastics threads or a fabric or felt strip or piece made of plastics threads are embedded in the narrow sides of the sleeve in the wall thereof.

18. A device according to any of claims 1 to 17, characterised in that an outwardly closed chamber enclosing at least one externally movable member, preferably a ball (15), is provided on at least one narrow side of the slit-like through opening (5).

19. A device according to claim 18, characterised in that in the direction of the through opening, the chamber had a seat (17) which receives and engages a ball (15) so as to obtain a permanently open position for the organ.

20. A device according to claim 19, characterised in that the ball chamber extends approximately in the plane of the slit and transversely to the direction of flow in the tubular body organ (Fig. 9).

21. A device according to claim 19, characterised in that the ball chamber extends approximately in the plane of the slit and parallel to the direction of flow in the tubular body organ (Fig. 10).

22. A device according to claim 21, characterised in that the ball chamber (41) has a seat (17) for a ball (15) at each end (Fig. 22).

23. A device according to any of claims 19 to 22, characterised in that the ball chamber contains a lubricant.

24. A device according to any of claims 1 to 17, characterised in that a chamber (33) closed by a thin skin (32) from the through opening (5) is provided in at least one wall (1) thereof and a member, more particularly a pin or ball (15), is movable in the chamber so as to alter the width of the slit in the through opening (5) (Fig. 19).

25. A device according to claim 24, characterised in that the chamber (33) has at least one position (34) for engaging the member (15).

26. A device according to any of claims 18 to 25, characterised in that the movable member (15) has remote operating means (24 to 30) similar to a remote release for a camera (Figs. 15 to 19).

27. A device according to claim 28, characterised in that the remote operating means comprises a rigid plastics tube (25) embedded in silicone rubber and in which an externally movable element (24) for actuating the member (15) is slidably mounted.

28. A device according to any of claims 18 to 27, characterised in that the movable member, more particularly a ball (15), is made of polytetrafluorethylene.

29. A device according to any of claims 1 to 17, characterised in that a closed pressure chamber (42) is disposed on at least one narrow side of the slit-like through opening (5) and is connected to a pump device, and the pressure chamber (42) and the pump device are filled with a pressure fluid (Fig. 23).

30. A device according to claim 29, characterised in that the pressure chamber (42) is connected to the pump device by a tube (43).

31. A device according to any of the preceding claims, characterised in that the sleeve (1, 2) is made of silicone rubber.

**Revendications**

1. Dispositif implantable destiné sélectivement à l'ouverture et à l'obturation au moins partielle d'organes corporels tubulaires, en particulier de l'urètre, comprenant un manchon comportant un élastomère synthétique et présentant une ouverture en forme de fente pour le passage de l'organe, la largeur de l'ouverture de passage destinée à obtenir la position d'ouverture ou de fermeture étant modifiable par déformation du manchon,
    caractérisé en ce que,
    le manchon (1, 2) est élastique et déformable de la position d'ouverture ou de fermeture représentant la position de repos à la position de fermeture ou d'ouverture avec apparition d'une force élastique de rappel.

2. Dispositif selon la revendication 1, caractérisé en ce que le manchon se compose de deux demi-coquilles (1, 2) reliées par une charnière (3) et dont les extrémités libres (6 ; 8 ; 35, 37) sont destinées à être réunies après mise en place sur l'organe.

3. Dispositif selon la revendication 2, caractérisé en ce que la charnière est formée d'un amincissement (3) du manchon (1, 2), par exemple sous la forme d'une charnière constituée d'un film.

4. Dispositif selon la revendication 2 ou 3, caractérisé en ce que les extrémités libres du manchon (1, 2) sont formées de pattes (6) se cousant l'une sur l'autre.

5. Dispositif selon la revendication 2 ou 3, caractérisé en ce que chacune des extrémités libres comporte du côté extérieur une rainure (9) de logement d'une agrafe de liaison (10) ou d'un anneau élastique.

6. Dispositif selon la revendication 2 ou 3, caractérisé en ce qu'une fermeture à aimants (40) est disposée aux extrémités libres du manchon (1, 2) (figure 21).

7. Dispositif selon la revendication 2 ou 3, caractérisé en ce qu'une fermeture à boutons-pression est disposée aux extrémités libres du manchon.

8. Dispositif selon la revendication 2 ou 3, caractérisé en ce que l'une des extrémités libres du manchon (2) comporte un prolongement (35) en forme de bande comportant des ergots ou des bourrelets (38) formant des crans d'arrêt et l'autre extrémité libre (37) comporte un passage (36) de forme complémentaire de celle du prolongement en forme de bande (figure 20).

9. Dispositif selon l'une des revendications 1 à 8, caractérisé en ce que le manchon (1, 2) a une section circulaire ou ovale et comporte sur le côté intérieur des coussins (4) remplis de gel et délimitant entre eux l'ouverture de passage (5) en forme de fente (figures 1 à 4).

10. Dispositif selon l'une des revendications 1 à 8, caractérisé en ce que le manchon (1, 2) a une section sensiblement rectangulaire et comprend deux cloisons disposées face à face et délimitant entre elles l'ouverture de passage (5) en forme de fente.

11. Dispositif selon la revendication 10, caractérisé en ce que les côtés étroits du manchon qui sont orientés parallèlement à l'ouverture de passage (5) comportent des creux de manipulation (14).

12. Dispositif selon l'une des revendications 1 à 11, caractérisé en ce que l'ouverture de passage (5) est élargie en forme de trémie (13, 31) du côté de l'entrée par rapport au sens de l'écoulement (figures 8, 16).

13. Dispositif selon l'une des revendications 1 à 12, caractérisé en ce qu'une cloison (11) de délimitation de l'ouverture de passage (5) comporte au moins un bourrelet (11) orienté transversalement par rapport à l'ouverture de passage (figure 8).

14. Dispositif selon la revendication 13, caractérisé en ce qu'une rainure (12) faisant face au bourrelet (11) est réalisée dans l'autre cloison (2) de délimitation de l'ouverture de passage (figure 8).

15. Dispositif selon l'une des revendications 1 à 14, caractérisé en ce que des fils de matière synthétique ou un ruban ou une pièce (18) de tissu ou de feutre constitué de fils de matière synthétique est ou sont disposés sur le manchon (1, 2) (figures 9, 13).

16. Dispositif selon la revendication 15, caractérisé en ce que les fils de matière synthétique ou un ruban ou une pièce (18) de tissu ou de feutre formé de fils de matière synthétique est ou sont enrobés au moins sur un côté de l'ouverture de passage dans la cloison du manchon (1, 2) (figures 9, 13).

17. Dispositif selon la revendication 15, caractérisé en ce que les fils de matière synthétique ou un ruban ou une pièce de tissu ou de feutre formé de fils de matière synthétique est ou sont enrobés sur les côtés étroits du manchon, dans la cloison de ce dernier.

18. Dispositif selon l'une des revendications 1 à 17, caractérisé en ce qu'une chambre fermée vers l'extérieur est prévue au moins sur un côté étroit de l'ouverture de passage (5) en forme de fente et renferme au moins un corps, de préférence une bille (15) déplaçable de l'extérieur.

19. Dispositif selon la revendication 18, caractérisé en ce que la chambre comporte vers l'ouverture de passage un siège (17) qui loge une bille (15) en constituant un cran d'arrêt de manière à permettre d'obtenir une position d'ouverture permanente de l'organe.

20. Dispositif selon la revendication 19, caractérisé en ce que la chambre de la bille est orientée approximativement dans le plan de la fente et transversalement par rapport à la direction d'écoulement dans l'organe tubulaire du corps (figure 9).

21. Dispositif selon la revendication 19, caractérisé en ce que la chambre de la bille est orientée approximativement dans le plan de la fente et parallèlement à la direction d'écoulement dans l'organe tubulaire du corps (figure 10).

22. Dispositif selon la revendication 21, caractérisé en ce que la chambre à bille (41) comporte à chacune des deux extrémités un siège (17) dont chacun est destiné à une bille (15) (figure 22).

23. Dispositif selon l'une des revendications 19 à 22, caractérisé en ce que la chambre à bille contient un lubrifiant.

24. Dispositif selon l'une des revendications 1 à 17, caractérisé en ce qu'une chambre (33) fermée par une pellicule mince (32) qui la sépare de l'ouverture de passage (5) est prévue au moins dans une cloison (1) de cette dernière et renferme un corps, en particulier une cheville ou une bille (15) qui est déplaçable de manière à faire varier la largeur de la fente de l'ouverture de passage (5) (figure 19).

25. Dispositif selon la revendication 24, caractérisé en ce que la chambre (33) comporte au moins un cran d'arrêt (34) destiné audit corps (15).

26. Dispositif selon l'une des revendications 18 à 25, caractérisé en ce que le corps déplaçable (15) est muni d'une télécommande (24 à 30) du type d'un déclencheur à distance d'une caméra photographique (figures 15 à 19).

27. Dispositif selon la revendication 26, caractérisé en ce que la télécommande comprend un tube de matière synthétique rigide enrobé dans du caoutchouc au silicone et dans lequel est monté à glissement un organe d'actionnement (24) dont l'action s'exerce sur ledit corps (15) et qui est déplaçable de l'extérieur.

28. Dispositif selon l'une des revendications 18 à 27, caractérisé en ce que le corps déplaçable, en particulier une bille (15), est en polytétrafluoréthylène.

29. Dispositif selon l'une des revendications 1 à 17, caractérisé en ce qu'une chambre fermée de compression (42), qui est disposée au moins sur un côté étroit de l'ouverture de passage en forme de fente (5), communique avec un dispositif de pompage et en ce que la chambre de compression (42) et le dispositif de pompage sont remplis d'un liquide sous pression (figure 23).

30. Dispositif selon la revendication 29, caractérisé en ce que la chambre de compression (42) communique par un tube (43) avec le dispositif de pompage.

31. Dispositif selon l'une des revendications précédentes, caractérisé en ce que le manchon (1, 2) est en caoutchouc au silicone.

FIG.1

FIG. 2

FIG.3

FIG.4

FIG.5

FIG.6

FIG.7

FIG.8

FIG.9

FIG.10

FIG.11

FIG.12

FIG.13

FIG.14

FIG.15

FIG.16

FIG.17

FIG.18

FIG.19

FIG. 20

FIG. 21

FIG. 22

FIG. 23